(19) Europäisches Patentamt
European Patent Office
Office européen des brevets

(11) Veröffentlichungsnummer: **0 014 379**
**B1**

# (12) EUROPÄISCHE PATENTSCHRIFT

(45) Veröffentlichungstag der Patentschrift: **02.06.82**

(51) Int. Cl.³: **C 07 C 125/073**

(21) Anmeldenummer: **80100338.5**

(22) Anmeldetag: **23.01.80**

(54) **Verfahren zur Herstellung von Methylen-bis-(4-phenyl-carbaminsäureester).**

(30) Priorität: **09.02.79 DE 2904917**

(43) Veröffentlichungstag der Anmeldung: **20.08.80 Patentblatt 80/17**

(45) Bekanntmachung des Hinweises auf die Patenterteilung: **02.06.82 Patentblatt 82/22**

(84) Benannte Vertragsstaaten: **AT BE DE FR GB IT NL**

(56) Entgegenhaltungen:
**US-A-2 015 039**
**Houben-Weyl, Methoden der organischen Chemie (4. Aufl.), Band 6/3, Seiten 287, 318**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Rubensstrasse 25, D-6700 Ludwigshafen (DE)**

EP 0 014 379 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Methylen-bis-(4-phenylcarbaminsäureester)

Diese Erfindung betrifft ein neues Verfahren zur Herstellung von Methylen-bis-(4-phenylcarbaminsäureester) durch Umsetzung von N-Phenylcarbaminsäureester mit Formaldehydacetalen.

Methylen-bis-(4-phenylcarbaminsäureester) sind wertvolle Ausgangsstoffe für die Herstellung von Methylen-bis-(4-phenylisocyanaten), die bekanntlich für die Herstellung von Polyurethanen Verwendung finden (s. DE-OS 2 635 490).

Zur Herstellung der Methylen-bis-(4-phenylcarbaminsäureester) kondensiert man Anilin mit Formaldehyd in Gegenwart von Salzsäure und setzt das so erhältliche Methylen-bis-(4-anilin) in Gegenwart von Basen mit Chlorameisensäureester um oder man stellt aus Methylen-bis-(4-anilin) und Phosgen Methylen-bis-(4-phenylisocyanat) her, das man dann mit den entsprechenden Alkoholen umsetzt.

Diese Verfahren haben den Nachteil, daß bei der Kondensation von Anilin mit Formaldehyd in hohem Umfang Oligomeren- und Isomerengemische entstehen und die Umsetzung mit Phosgen aus Sicherheitsgründen eine aufwendige Technik erfordert. Außerdem belastet die zur Herstellung von Methylen-bis-(4-anilin) benötigte Salzsäure und die bei der weiteren Umsetzung zusätzlich anfallende Salzsäure die Umwelt.

Methylen-bis-(4-phenylcarbaminsäureester) lassen sich auch durch Umsetzung von Methylen-bis-(4-nitrophenyl) mit Alkoholen und Kohlenmonoxid herstellen (s. DE-AS 1 568 044). Da die Darstellung der hierfür benötigten Nitroverbindung große Schwierigkeit bereitet, hat dieses Verfahren keine technische Bedeutung erlangt.

Es wurde nun gefunden, daß man Methylen-bis-(4-phenylcarbaminsäureester) besonders vorteilhaft und in ausgezeichneter Ausbeute erhält, wenn man N-Phenylcarbaminsäureester in Gegenwart einer Säure mit Formaldehydacetalen bei Temperaturen bis 200° C umsetzt.

Das Reaktionsschema kann für den Fall der Bildung des Methylen-bis-(4-phenylcarbaminsäuremethylesters) durch folgende Formeln wiedergegeben werden:

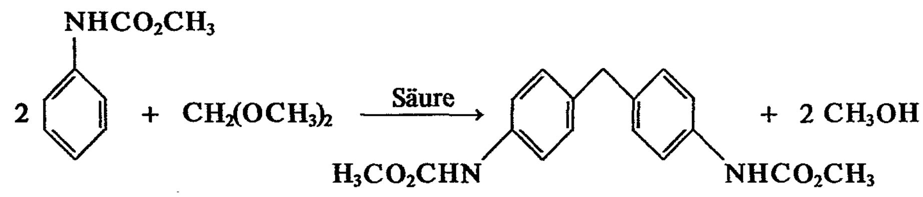


Als N-Phenylcarbaminsäureester kommen z. B. Verbindungen der Formel

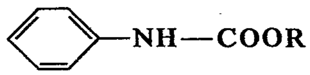

in Betracht, in der R ein Alkylrest mit 1 bis 4 C-Atomen bedeutet und der Phenylrest in den o- und/oder m-Stellungen durch Methylgruppen substituiert sein kann.

Geeignete N-Phenylcarbaminsäureester sind beispielsweise N-Phenylcarbaminsäuremethyl-, äthyl-, propyl- oder butylester, N-o-Tolylcarbaminsäuremethyl- oder äthylester, N-2,6-Dimethylphenylcarbaminsäuremethyl- oder äthylester oder N-m-Tolylcarbaminsäuremethylester.

Als Formaldehydacetale verwendet man z. B. Verbindungen der Formel

$$CH_2(OR)_2$$

in der R ein Alkylrest mit 1 bis 4 C-Atomen bedeutet. Vorzugsweise werden jeweils solche Acetale gewählt, in denen der Alkylrest mit dem Alkanol übereinstimmt, der den Carbaminsäureester gebildet hat.

Von besonderem technischen Interesse ist die erfindungsgemäße Herstellung der Methylen-bis-(4-phenylcarbaminsäureester) aus dem Methyl- oder dem Äthylester der N-Phenylcarbaminsäure und dem Dimethyl- oder Diäthylacetal des Formaldehyds.

Man führt die Umsetzung der Ausgangsstoffe in Gegenwart einer Säure bei Temperaturen bis 200° C, vorzugsweise bei 50 bis 150° C, insbesondere 70 bis 130° C durch. Dabei liegt das Molverhältnis Carbaminsäureester: Acetal z. B. bei 1 : 1 bis 1 : 0,1, vorzugsweise bei 1 : 0,6 bis 1 : 0,2.

Als Säuren, die man z. B. in Mengen von 1 bis 100, vorzugsweise 10 bis 60 Mol-%, bezogen auf den Carbaminsäureester, anwendet, sind z. B. Phosphorsäure, Schwefelsäure, Alkylsulfonsäure wie Methansulfonsäure oder Arylsulfonsäure wie p-Toluolsulfonsäure geeignet. Entsprechend einer besonders vorteilhaften Ausführungsform der Erfindung verwendet man eine starke Säure, die vom Reaktionsgemisch destillativ abgetrennt werden kann, beispielsweise Trifluormethansulfonsäure. Auf diese Weise entfällt die Aufarbeitung des Reaktionsgemisches mit Wasser oder Basen und die Säure kann direkt erneut der Reaktion zugeführt werden.

Nach einer weiteren vorteilhaften Ausführungsform der Erfindung verwendet man als Säuren stark saure organische Kationenaustauscher, beispielsweise sulfonsaure Austauscherharze. Diese Ionenaustauscher werden nach an sich bekannten Methoden entweder im Reaktionsgemisch suspendiert oder in einem Festbett angeordnet.

Man führt das erfindungsgemäße Verfahren vorzugsweise unter möglichst weitgehender Abwesenheit von Wasser, insbesondere in Abwesenheit von Wasser, d. h. unter Verwendung von Säuren durch, die praktisch kein Wasser enthalten. Man kann sowohl ohne als auch in Gegenwart eines nicht wäßrigen Lösungsmittels, beispielsweise Benzol, Methylcyclohexan, Essigsäure, Methanol, Methylacetat, Nitrobenzol, Chlorbenzol, Dichlorbenzol oder chlorierten aliphatischen Kohlenwassestoffen arbeiten.

Die Umsetzung, die etwa nach 0,5 bis 20 Stunden beendet ist, wird im allgemeinen so durchgeführt, daß man entweder zu einem Gemisch aus dem Carbaminsäureester und der Säure unter Rühren bei der Reaktionstemperatur das Acetal langsam zugibt oder daß man ein Gemisch aus dem Carbaminsäureester, dem Acetal und der Säure unter Rühren erhitzt und die Gemische entsprechende Zeit auf der Reaktionstemperatur hält. Die Isolierung des Reaktionsproduktes erfolgt nach herkömmlichen Methoden, z. B. durch Extraktion der Säure mit Wasser oder Neutralisation mit einer Base. Das gegebenenfalls vorhandene Lösungsmittel und unumgesetzte Ausgangsprodukte werden mittels einer Vakuumdestillation abgetrennt.

Die Kondensation der Phenylcarbaminsäureester mit den erwähnten Formaldehydderivaten kann in Einzelansätzen oder als kontinuierliches Verfahren durchgeführt werden.

Die US-PS 2 015 039 enthält Angaben über die Bildung von Diaminodiphenylmethan durch Erhitzen von Methylendiethylether mit Anilin. Daß diese Reaktion anders verläuft, ist aus Houben Weyl, Methoden der Organischen Chemie, 4. Auflage Band 6/3, Seite 318, zu schließen, wo angegeben ist, daß sich Acetale mit aromatischen Aminen zu Iminen umsetzen. Es war somit nicht zu erwarten, daß Methylen-bis-(4-phenylcarbaminsäureester) nach dem erfindungsgemäßen Verfahren in so hoher Ausbeute erhalten werden.

Aus der US-PS 2 946 768 und der DE-PS 1 042 891 ist bekannt, daß man Phenylcarbaminsäureester mit Carbonylverbindungen, wie Formaldehyd in Gegenwart von Säuren, wie Salzsäure oder Schwefelsäure zu Kondensationsprodukten umsetzen kann. Es ist angegeben, daß die Art der gebildeten Kondensationsprodukte ungewiß ist. So wird eine C-C Verknüpfung für wahrscheinlich gehalten, eine N-C Verknüpfung aber nicht ausgeschlossen (s. US-PS 2 946 768, Spalte 2, Zeilen 64 bis 70). In der DE-OS 2 832 379, Seite 3, Zeilen 1 bis 19, wird mitgeteilt, daß bei dem in der US-PS 2 946 768 beschriebenen Verfahren die Carbonylverbindung zur Reaktion mit dem Stickstoffatom des Carbamats neigt, so daß 15 bis 20 Gewichtsprozent an unerwünschten Produkten mit N-C Verknüpfung entstehen; außerdem sollen noch andere Nebenprodukte gebildet werden.

Da es kein Verfahren zur Abtrennung der über das Stickstoffatom verknüpften Produkte, die bei der Pyrolyse keine Isocyanaten liefern, gibt, sind die nach der US-PS 2 946 768 und der DE-PS 1 042 891 hergestellten Reaktionsgemische zur Herstellung von Isocyanaten ungeeignet. Dieses Verfahren ist daher in technischer und ökonomischer Hinsicht unbefriedigend.

In der DE-OS 2 832 379 wird nun ein Verfahren zur Umlagerung dieser unerwünschten Nebenprodukte zu Methylen-bis-phenylcarbaminsäureestern und davon abgeleiteten höheren homologen Polymethylenpolyphenylcarbaminsäureestern beschrieben, bei dem man die nach dem Verfahren der US-PS 2 946 768 hergestellten Reaktionsgemische unter praktisch wasserfreien Bedingungen bei 50 bis 170°C mit starken Protonensäuren oder Lewissäuren umsetzt. Dieses zweistufige Verfahren ist technisch schwierig und umständlich, auch wirft es erhebliche Abwasserprobleme auf.

Ein wesentlicher Unterschied, durch den sich das erfindungsgemäße Verfahren von den Verfahren der US-PS 2 946 768 und DE-OS 2 832 379, bei denen Formaldehyd oder Formaldehyd bildende Verbindungen verwendet werden, so daß stets Wasser im Reaktionsgemisch vorliegt, unterscheidet, besteht darin, daß bei dem erfindungsgemäßen Verfahren kein Formaldehyd und somit kein Reaktionswasser gebildet wird. Eine Hydrolyse der Carbaminsäureester zu Aminen, die Bildung von Harnstoffen und die damit verbundene Belastung des Abwassers werden somit unterbunden.

## Beispiel 1 (Vergleichsbeispiel)

Analog Beispiel 2 der DE-PS 1 042 891 wird ein Gemisch aus 183 Teilen Phenylcarbaminsäuremethylester, 500 ml Wasser und 86 Teilen Formalin (30%ig) unter Rühren auf 100°C erwärmt. Dann fügt man 100 ml konzentrierte Salzsäure hinzu. Das Reaktionsgemisch wird anschließend 20 Stunden bei 100°C gerührt. Nach Beendigung der Umsetzung trennt man die wäßrige Phase ab. Das Reaktionsprodukt wird dreimal mit heißem Wasser gewaschen, anschließend wird im Vakuum unumgesetztes Ausgangsprodukt abdestilliert. Der Rückstand wird mittels Hochdruck-Flüssigkeits-Chromatographie (HPLC) analysiert. Er enthält 50% Methylen-bis-phenylcarbaminsäuremethylester, 9% Dreikernprodukt, 16% N-C-verknüpftes Zweikernprodukt und 10% N-C-verknüpftes Dreikernprodukt. Der Rest besteht aus nicht näher identifizierten höherkernigen Verbindungen.

Beispiel 2 (Vergleichsbeispiel)

Ein Gemisch aus 90 Teilen Phenylcarbaminsäuremethylester, 250 ml Chlorbenzol und 40 Teilen Formalin (30%ig) wird unter Rühren auf 100° C erwärmt, dann fügt man 50 ml konzentrierte Salzsäure hinzu. Das Reaktionsgemisch wird anschließend 20 Stunden bei 100° C gerührt. Nach Beendigung der Umsetzung wird die wäßrige Phase abgetrennt und mit Wasser zweimal gewaschen. Anschließend werden Chlorbenzol und unumgesetztes Ausgangsprodukt abdestilliert. Entsprechend der HPLC-Analyse enthält der Rückstand 49% Methylen-bis-phenylcarbaminsäuremethylester, 12% Dreikernprodukt, 15% N-C-verknüpftes Zweikern- und 9% Dreikernprodukt. Der Rest besteht aus nicht näher identifizierten höherkernigen Verbindungen.

Beispiel 3

In einem Rührautoklaven wird ein Gemisch aus 151 Teilen Phenylcarbaminsäuremethylester, 38 Teilen Dimethylformal, 120 Teilen Nitrobenzol und 50 Teilen ®LEWATIT SPC-108 unter Rühren auf 120° C erhitzt und 15 Stunden bei dieser Temperatur gerührt. Nach Beendigung der Umsetzung wird der Katalysator abgetrennt. Dann werden im Vakuum Nitrobenzol und nicht umgesetztes Ausgangsmaterial abdestilliert. Man erhält 142 Teile eines Destillationsrückstandes, der entsprechend der HPLC-Analyse aus 56% Methylen-bis-phenylcarbaminsäuremethylester, 23% 3-Kernprodukt und 21% höherkernigen Produkten besteht.

LEWATIT SPC-108 ist ein im Handel erhältlicher makroporöser sulfonsaurer Kationenaustauscher aus Styrol-Di-vinylbenzol (8%)-Co-polymerisat mit einer Korngröße von 0,3—1,5 mm, einer Totalkapazität von etwa 4,2 mval/g Trockensubstanz, einer Porenoberfläche von ca. 20 $m^2$/g (nach BET) und einem mittleren Porendurchmesser von etwa 500 Å (nach BET).

Beispiel 4

In einem Rührautoklaven wird ein Gemisch aus 227 Teilen Phenylcarbaminsäuremethylester, 38 Teilen Dimethylformal und 60 Teilen LEWATIT SC-104 unter Rühren auf 120° C erhitzt und 15 Stunden bei dieser Temperatur gerührt.

Nach Beendigung der Umsetzung wird der Katalysator abgetrennt. Dann wird im Vakuum nicht umgesetztes Ausgangsprodukt abdestilliert. Man erhält 136 Teile eines Destillationsrückstandes, der entsprechend der HPLC-Analyse aus 68% Methylen-bis-phenylcarbaminsäuremethylester, 20% 3-Kernprodukt und 12% höherkernigen Produkten besteht.

LEWATIT SC-104 ist ein im Handel erhältlicher gelartiger sulfonsaurer Kationenaustauscher aus Styrol-Divinylbenzol-(4%)-Copolymerisat mit einer Korngröße von 0,3—1,2 mm und einer Totalkapazität von etwa 4,2 mval/g Trockensubstanz.

Beispiel 5

In einem Rührautoklaven wird ein Gemisch aus 151 Teilen Phenylcarbaminsäuremethylester, 38 Teilen Dimethylformal, 110 Teilen Chlorbenzol und 20 Teilen Trifluormethansulfonsäure unter Rühren auf 100° C erhitzt und 10 Stunden bei dieser Temperatur gerührt. Nach Beendigung der Umsetzung werden im Vakuum der Katalysator, das Lösungsmittel und nicht umgesetztes Ausgangsmaterial abdestilliert.

Man erhält 150 Teile eines Destillationsrückstandes, der entsprechend der HPLC-Analyse aus 52% Methylen-bis-phenylcarbaminsäuremethylester, 30% 3-Kernprodukt und 18% höherkernigen Produkten besteht.

Beispiel 6

In einem Rührautoklaven wird ein Gemisch aus 375 Teilen Phenylcarbaminsäuremethylester, 150 Teilen Nitrobenzol, 38 Teilen Dimethylformal und 30 Teilen konzentrierter Schwefelsäure unter Rühren auf 100° C erhitzt und 15 Stunden bei dieser Temperatur gerührt. Nach Beendigung der Umsetzung und Extraktion der Säure mit Wasser werden im Vakuum das Lösungsmittel und nicht umgesetztes Ausgangsmaterial abdestilliert. Der Destillationsrückstand wird in Toluol umkristallisiert. Man erhält 110 Teile Methylen-bis-(4-phenylcarbaminsäuremethylester).

Beispiel 7

In einem Rührreaktor wird eine Suspension aus 151 Teilen (1 Mol) N-Phenylcarbaminsäuremethylester, 38 Teilen (0,5 Mol) Formaldehyddimethylacetal und 30 g des unter der Bezeichnung LEWASORB AC 10 im Handel erhältlichen sulfonsauren Kationenaustauschers mit der Korngröße 10 bis 200 μ unter Rühren auf 80° C erhitzt und zwei Stunden bei dieser Temperatur gerührt. Der Katalysator wurde vor der Verwendung 20 Stunden bei 100° C im Vakuum getrocknet. Nach Beendigung der Reaktion werden der Katalysator abfiltriert und die nicht umgesetzten Ausgangsstoffe abdestilliert. Durch Umkristallisation des Rückstandes aus Toluol erhält man 114 Teile reines Methylen-bis-(4-phenylcarbaminsäuremethylester), das sind 73% der Theorie.

Beispiel 8

In einem Rührautoklav wird eine Suspension aus 330 Teilen (2 Mol) N-Phenylcarbaminsäureäthylester, 45 Teilen (0,5 Mol) Formaldehyddiäthylacetal und 50 g des unter der Bezeichnung LEWASORB AC 10 im Handel erhältlichen sulfonsauren Kationenaustauschers mit der Korngröße 10 bis 200 μ unter Rühren auf 100° C erhitzt und zwei Stunden bei dieser Temperatur gerührt. Der Katalysator wurde vor der Verwendung 20 Stunden bei 100° C im Vakuum getrocknet. Nach Beendigung der Reaktion werden der Katalysator abfiltriert und die nicht umgesetzten Ausgangsstoffe abdestilliert. Durch Umkristallisation des Rückstandes aus Toluol erhält man 150 Teile reines Methylen-bis-(4-phenylcarbaminsäureäthylester), das sind 80% der Theorie.

**Patentansprüche**

1. Verfahren zur Herstellung von Methylen-bis-(4-phenylcarbaminsäureester), dadurch gekennzeichnet, daß man N-Phenylcarbaminsäureester in Gegenwart einer Säure mit Formaldehydacetalen bei Temperaturen bis 200° C umsetzt.

2. Verfahren nach Anspruch 1, dadurch gekennzeichnet, daß man als N-Phenylcarbaminsäureester eine Verbindung der Formel

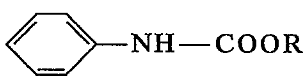

in der R ein Alkylrest mit 1 bis 4 C-Atomen bedeutet und der Phenylrest in den o- und/oder m-Stellungen durch Methylgruppen substituiert sein kann, verwendet.

3. Verfahren nach Anspruch 1, dadurch gekennzeichnet, daß man ein Formaldehydacetal der Formel

$CH_2(OR)_2$

in der R ein Alkylrest mit 1 bis 4 C-Atomen bedeutet, verwendet.

4. Verfahren nach Anspruch 1, dadurch gekennzeichnet, daß man N-Phenylcarbaminsäuremethylester oder N-Phenylcarbaminsäureäthylester mit Formaldehyddimethylacetal oder Formaldehyddiäthylacetal umsetzt.

5. Verfahren nach Anspruch 1, dadurch gekennzeichnet, daß man als Säure Trifluormethansulfonsäure verwendet.

6. Verfahren nach Anspruch 1, dadurch gekennzeichnet, daß man als Säuren stark saure Kationenaustauscher verwendet.

**Claims**

1. A process for the preparation of methylene-bis-(4-phenylcarbamic acid esters), characterized in that an N-phenyl-carbamic acid ester is reacted with a formaldehyde-acetal in the presence of an acid at up to 200° C.

2. A process as claimed in claim 1, characterized in that the phenylcarbamic acid ester used is a compound of the formula

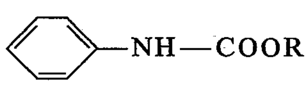

where R is alkyl of 1 to 4 carbon atoms and the phenyl radical may be substituted by methyl in the o- and/or m-positions.

3. A process as claimed in claim 1, characterized in that a formaldehyde-acetal of the formula

$$CH_2(OR)_2$$

where R is alkyl of 1 to 4 carbon atoms, is used.

4. A process as claimed in claim 1, characterized in that methyl N-phenylcarbamate or ethyl N-phenylcarbamate is reacted with formaldehyde-dimethylacetal or formaldehyde-diethylacetal.

5. A process as claimed in claim 1, characterized in that trifluoromethanesulfonic acid is used as the acid.

6. A process as claimed in claim 1, characterized in that a strongly acid cation exchanger is used as the acid.

**Revendications**

1. Procédé de préparation de méthylène-bis-(4-phényl-carbamates), caractérisé en ce que l'on fait réagir un N-phényl-carbamate à une température pouvant aller jusqu'à 200°C et en présence d'un acide avec un acétal du formaldéhyde.

2. Procédé suivant la revendication 1, caractérisé en ce que le N-Phényl-carbamate employé est un composé de la formule

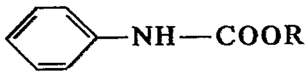

dans laquelle R désigne un radical alcoyle en $C_1$ à $C_4$ et dont le noyau phényle peut être substitué en position ortho et(ou) méta par des radicaux méthyle.

3. Procédé suivant la revendication 1, caractérisé en ce que l'on emploie un acétal du formaldéhyde de la formule

$$CH_2(OR)_2,$$

dans laquelle R désigne un radical alcoyle en $C_1$ à $C_4$.

4. Procédé suivant la revendication 1, caractérisé en ce que l'on fait réagier le N-phényl-carbamate de méthyle ou le N-phényl-carbamate d'éthyle avec l'acétal diméthylique du formaldéhyde ou avec l'acétal diéthylique du formaldéhyde.

5. Procédé suivant la revendication 1, caractérisé en ce que l'acide utilisé est l'acide trifluorométhanesulfonique.

6. Procédé suivant la revendication 1, caractérisé en ce que l'on emploie en tant qu'acide un échangeur de cations fortement acide.